Europäisches Patentamt

European Patent Office

Office européen des brevets

(11) Veröffentlichungsnummer: **0 007 570**
**B1**

(12)                          **EUROPÄISCHE PATENTSCHRIFT**

(45) Veröffentlichungstag der Patentschrift:
11.07.84

(51) Int. Cl.³: **C 07 C 47/127, C 07 C 45/29**

(21) Anmeldenummer: **79102523.2**

(22) Anmeldetag: **18.07.79**

(54) Verfahren zur Herstellung von Glyoxal aus Äthylenglykol.

(30) Priorität: 24.07.78 DE 2832405

(43) Veröffentlichungstag der Anmeldung:
06.02.80 Patentblatt 80/3

(45) Bekanntmachung des Hinweises auf die Patenterteilung:
11.07.84 Patentblatt 84/28

(84) Benannte Vertragsstaaten:
BE DE FR GB IT NL

(56) Entgegenhaltungen:
DE - A - 2 634 439
GB - A - 1 272 592

Die Akte enthält technische Angaben, die nach dem
Eingang der Anmeldung eingereicht wurden und die
nicht in dieser Patentschrift enthalten sind.

(73) Patentinhaber: **BASF Aktiengesellschaft,
Carl-Bosch-Strasse 38, D-6700 Ludwigshafen (DE)**

(72) Erfinder: **Engelbach, Heinz, Dr., Kropsburgstrasse 24,
D-6703 Limburgerhof (DE)**
Erfinder: **Sprague, Michael Jolyon, Dr. Chem., Lachner
Strasse 3, D-6800 Mannheim 1 (DE)**

## Beschreibung

Aus den U.S.-Patentschriften 2 339 282 und 2 339 346 bis 2 339 348 ist bekannt, dass man aus Äthylenglykol durch Dampfphasenoxidation in Gegenwart von Sauerstoff an Katalysatoren auf Basis von Kupfer oder Kupferoxid bei Temperaturen von 225 bis 450°C Glyoxal herstellen kann. Setzt man den kupferhaltigen Katalysatoren entsprechend den Angaben der DT-OS 1 923 048 Germanium, Zinn, Blei, Stickstoff, Posphor, Arsen, Antimon oder Wismut zu, so werden Glyoxalausbeuten bis zu 72% erzielt.

Ein erheblicher Nachteil bei diesen bekannten Verfahren ergibt sich aus der Tatsache, dass die Katalysatoren relativ rasch altern, so dass die Ausbeute alsbald abfällt. So wurde festgestellt, dass die Glyoxalausbeute bei Verwendung von Kupferdrehspänen als Katalysator bei 310°C und kontinuierlichem Betrieb schon nach einer 73 stündigen Laufzeit von ca. 68 auf ca. 59% abfällt. Wird ein in der DT-OS 1 923 048 beschriebener Katalysator für die Oxidation von Äthylenglykol zu Glyoxal herangezogen, so fällt nach den Angaben der DT-OS 2 158 343 die Katalysatorselektivität bei einer 700stündigen Betriebsdauer von einem Anfangswert von 60 bis 64% auf 55%. Aus DT-OS 2 158 344 geht hervor, dass nach 18 Tagen Betriebsdauer die Selektivität eines in der DT-OS 1 923 048 beschriebenen Katalysators von anfänglich 58 auf 54% gesunken ist. Um kupferhaltige Katalysatoren zu regenerieren, hat man vorgeschlagen, sie unter Unterbrechung der normalen Betriebsweise entweder oxidierend oder reduzierend zu behandeln. Nach den Angaben der DT-OS 2 158 343 wird ein CU-Ag-P-Katalysator nach 700 Stunden Laufzeit durch eine 12stündige Reduktion bei 450°C regeneriert. In der DT-OS 2 158 344 wird die Regenerierung eines CU-P-Katalysators nach einer 18 tägigen Laufzeit beschrieben, bei der über den Katalysator mindestens 1 Tag Sauerstoffüberschuss geleitet wird.

Diese Regenerierverfahren haben den Nachteil, dass sie mit einem Produktionsausfall verbunden sind und aufwendige Sicherheitsmassnahmen erfordern, um die Vermischung der zum Regenerieren verwendeten Luft mit dem Reaktionsgas auszuschliessen. Aus der DE-OS 2 634 439 ist bekannt, dass man bei der Gasphasenoxidation von Ethylenglykol an einem mit Posphor dotierten Katalysator aus Kupfer und/oder Silber die Glyoxalausbeute verbessern kann, wenn man zum gasförmigen Ausgangsgemisch flüchtige Bromverbindungen, wie Bromalkane, Brombenzole oder Bromwasserstoff gibt. Als geeignete Bromverbindung wird auch Phosphortribromid genannt. Wie sich gezeigt hat, werden auch bei Verwendung von Phosphortribromid sehr gute Glyoxalausbeuten erhalten. Allerdings setzt sehr bald ein zunächst langsamer, sich bei weiterem Betrieb denn aber schnell verstärkender Abfall der Ausbeute ein. Im übrigen stellt die Verwendung von flüchtigen Bromverbindungen aus Gründen der Korrosionsgefahr und der geforderten hohen Produktreinheit keine technisch befriedigende Alternative bei der Herstellung von Glyoxal durch Dampfphasenoxidation von Ethylenglykol dar.

Es wurde nun gefunden, dass man bei der Herstellung von Glyoxal durch Dampfphasenoxidation von Ethylenglykol mit einem Sauerstoff enthaltenden Gas in Gegenwart von Kupfer enthaltenden Oxidationskatalysatoren bei erhöhter Temperatur die Lebensdauer der Katalysatoren erheblich verlängern kann und auch bei längeren Betriebszeiten gleichbleibend hohe Ausbeuten erhält, wenn man die Dampfphasenoxidation in Gegenwart einer unter den Reaktionsbedingungen gasförmigen Phosphorverbindung, mit Ausnahme von anorganischen Phosphorverbindungen, durchführt, wobei die Phosphormenge (berechnet P), bezogen auf die Gewichtmenge an Äthylenglykol, 1 bis 100 ppm beträgt.

Überraschenderweise entfaltet Phosphor nur in Form der unter den Reaktionsbedingungen gasförmigen Verbindungen diese vorteilhafte Wirkung, wobei bereits sehr kleine P-Mengen genügen. Vorzugsweise beträgt die Phosphormenge (berechnet P), bezogen auf die Gewichtsmenge an Ethylenglykol, 2 bis 25 ppm.

Man wählt zweckmässig solche Phosphorverbindungen aus, die einen ähnlichen Siedepunkt wie Äthylenglykol haben. Geeignete Phosphorverbindungen sind z. B. Trimethylphosphat, Triäthylphosphat, Tri-iso-propylphosphat, Tri-n-propylphosphat, Trimethylphosphit, Triäthylphosphit, Triäthylphosphinoxid, Methylphosphonsäurediäthylester, Methylphosphonsäuredimethylester oder Äthylphosphonsäurediäthylester.

Die Dampfphasenoxidation des Äthylenglykols mit dem Sauerstoff enthaltenden Gas an den Kupfer enthaltenden Katalysatoren führt man auf an sich bekannte Weise, z. B. bei Temperaturen bei ca. 225° bis ca. 500°C, durch. Als Kupfer enthaltende Katalysatoren sind beispielsweise metallisches Kupfer, kupferhaltige Legierungen oder Verbindungen mit Metallen oder Nichtmetallen geeignet, wie Kupferphosphide, Kupferbronzen oder Legierungen des Kupfers mit Silber und/oder Gold, Kupfererze wie Malachit und Kupferverbindungen, die während der Reaktion ganz oder teilweise zu Kupfer reduziert werden können, wie Kupfer (I) oxid, Kupfer (II) oxid, und Verbindungen, die beim Erhitzen in Kupferoxide übergehen, wie Kupfernitrat und Kupferazetat. Ferner sind auch Kupferphosphat und Kupferantimonat geeignet. Den kupferhaltigen Verbindungen können zusätzlich noch andere Metall- oder Nichtmetalloxide wie die Oxide von Zink, Chrom, Phosphor, Antimon, Zinn und Wismut beigemischt sein. Die kupferhaltige katalytische Masse kann auch auf einem inerten Träger aufgebracht werden oder gegebenenfalls mit einem inerten Material verdünnt werden. Der Katalysator kann gegebenenfalls auch vor dem Einsatz einer reduzierenden Behandlung unterworfen werden.

Bevorzugt sind kupferhaltige Katalysatoren, die keine grosse innere Oberfläche besitzen, beispielsweise solche mit einer Oberfläche von unter 50 m² pro g. Besonderes technisches Interesse haben metallisches Kupfer und Legierungen, die Kupfer als einen wesentlichen Bestandteil enthalten. Man wendet sie z. B. in Form von Drehspänen, Drahtgeweben, Gazen oder auch als Trägerkatalysatoren mit einem z. B. oberflächenarmen, inerten Träger an.

Das Verfahren wird z. B. so ausgeführt, dass man ein gasförmiges Gemisch aus Äthylenglykol und Wasser (Wassergehalt 0,1 bis 99%) zusammen mit Sauerstoff in einer Menge zwischen ca. 0,5 und 2,0 Mol, bezogen auf 1 Mol eingesetztes Äthylenglykol, eventuell zusammen mit Stickstoff (0 bis 99 Volumen-% des Gesamtgasgemisches) über den auf 225 bis 500°C gehaltenen Katalysator leitet, wobei man die flüchtige Phosphorverbindung dem gasförmigen Ausgangsgemisch vorher zusetzt. Das den Reaktor verlassende gasförmige Produktgemisch wird wie üblich mit Wasser ausgewaschen.

Beispiel 1

25,25 Teile Kupferdrehspäne werden in einem Röhrenreaktor aus V2A-Stahl mit einem inneren Rohrdurchmesser von 21 mm so eingebaut, dass die Katalysatorfüllhöhe 18 cm beträgt. Man leitet kontinuierlich pro Stunde ein gasförmiges Gemisch aus 6,38 Gewichtsteilen Äthylenglykol, 2,04 Gewichtsteilen Wasser, 0,00019 Gewichtsteilen Trimethylphosphat (CH$_3$O)$_3$PO (das sind 6,58 ppm P, bezogen auf das Äthylenglykolgewicht), 3465 Volumenteilen Sauerstoff und 188 000 Volumenteilen Stickstoff über den auf einer Temperatur von 300°C gehaltenen Katalysator. Das Produktgemisch wird aus dem den Reaktor verlassenden Gasstrom mit Wasser ausgewaschen. Aufgrund der Analyse der erhaltenen wässrigen Lösung erhält man folgendes Ergebnis:

Eingesetzte Menge an Äthylenglykol
= 421,0 Gew.-Teile
Wässrige Produkt-Lösung = 1818,5 Gew.-Teile
Die Produkt-Lösung enthält (titrimetrisch bestimmt):
14,5 Gew. % Glyoxal = 263,7 Teile
(entspricht einer Glyoxalausbeute, bezogen auf eingesetztes Äthylenglykol, von 67,0% der Theorie).

Nach einer weiteren, ununterbrochenen Laufzeit von 96 Stunden werden folgende Ergebnisse erhalten:

Eingesetzte Menge an Äthylenglykol
= 45,32 Teile
Wässrige Produkt-Lösung = 537,99 Teile
Die Produktlösung enthält:
4,74 Gew.-% Glyoxal = 25,50 Teile
(entspricht einer Glyoxalausbeute, bezogen auf eingesetztes Äthylenglykol, von 60,2% der Theorie).

Vergleichsbeispiel (kein Zusatzt einer flüchtigen P-Verbindung).

Man verfährt wie in Beispiel 1 beschrieben, wobei man hier stündlich ein gasförmiges Gemisch aus 6,56 Gewichtsteilen Äthylenglykol, 2,10 Gewichtsteilen Wasser, 3570 Volumenteilen Sauerstoff und 188000 Volumenteilen Stickstoff über den auf einer Temperatur von 310°C gehaltenen Katalysator leitet.

Man erhält folgendes Ergebnis:
Eingesetzte Menge an Äthylenglykol
= 32,01 Gew.-Teile
Wässrige Produkt-Lösung = 360,42 Gew.-Teile
Die Produkt-Lösung enthält
5,61 Gew.-% Glyoxal = 20,22 Teile
(entspricht einer Glyoxalausbeute, bezogen auf eingesetztes Äthylenglykol, von 67,6% der Theorie).

Nach einer weiteren, ununterbrochenen Laufzeit von 73 Stunden werden folgende Ergebnisse erhalten:

Eingesetzte Menge an Äthylenglykol
= 40,41 Teile
Wässrige Produkt-Lösung = 451,72 Teile
Die Produkt-Lösung enthält:
4,90 Gew.-% Glyoxal = 22,13 Teile
(entspricht einer Glyoxalausbeute, bezogen auf eingesetztes Äthylenglykol, von 58,6% der Theorie).

Beispiel 2

In einem wie in Beispiel beschriebenen mit Kupferdrehspänen beschickten Röhrenreaktor wird kontinuierlich pro Stunde ein gasförmiges Gemisch aus 6,53 Teilen Äthylenglykol, 2,08 Teilen Wasser, 0,000389 Teilen Trimethylphosphat (CH$_3$O)$_3$PO (das sind 13,2 ppm P, bezogen auf das Äthylenglykolgewicht), 3465 Volumenteilen Sauerstoff und 188000 Volumenteilen Stickstoff über den bei 310°C gehaltenen Katalysator geleitet. Man arbeitet den den Reaktor verlassenden Gasstrom wie in Beispiel 1 beschrieben auf. Dabei wird folgendes Ergebnis erhalten:

Eingesetzte Menge an Äthylenglykol
= 38,84 Teile
Wässrige Produkt-Lösung = 560,33 Teile
Die Produktlösung enthält
4,40 Gew.-% Glyoxal = 24,65 Teile
(entspricht einer Glyoxalausbeute, bezogen auf eingesetztes Äthylenglykol, von 67,9% der Theorie).

Nach einer weiteren, ununterbrochenen Laufzeit von 1 166 Stunden wird folgendes Ergebnis erhalten:

Eingesetzte Menge an Äthylenglykol
= 37,43 Teile
Wässrige Produkt-Lösung = 508,86 Teile
Die Produkt-Lösung enthält
4,46 Gew.-% Glyoxal = 22,70 Teile
(entspricht einer Glyoxalausbeute, bezogen auf eingesetztes Äthylenglykol, von 64,8% der Theorie).

Vergleichsbeispiel (zuviel flüchtige P-Verbindung.

In einem wie in Beispiel 1 beschriebenen mit Kupferdrehspänen beschickten Röhrenreaktor wird kontinuierlich pro Stunde ein gasförmiges Gemisch aus 6,19 Teilen Äthylenglykol, 1,97 Teilen Wasser, 0,00367 Teilen Trimethylphosphat (CH$_3$O)$_3$PO (das sind 131 ppm P, bezogen auf das Äthylenglykolgewicht), 2645 Volumenteilen Sauerstoff und 185000 Volumenteilen Stickstoff über den bei 300°C gehaltenen Katalysator geleitet. Man arbeitet den den Reaktor verlassenen Gasstrom wie in Beispiel 1 beschrieben auf, wobei man folgendes Ergebnis erhält:

Eingesetzte Menge an Äthylenglykol
= 37,63 Teile
Wässrige Produkt-Lösung = 461,64 Teile
Die Produktlösung enthält:
5,20 Gew.-% Glyoxal = 24,01 Teile
(entspricht einer Glyoxalausbeute, bezogen auf eingesetztes Äthylenglykol, von 68,2% der Theorie).

Nach einer weiteren, ununterbrochenen Laufzeit von 48 Stunden erhält man folgendes Ergebnis:

Eingesetzte Menge an Äthylenglykol
= 37,02 Gew.-Teile
Wässrige Produkt-Lösung = 543,71 Gew.-Teile
Die Produkt-Lösung enthält:
3,49 Gew.-% Glyoxal = 18,98 Teile
(entspricht einer Glyoxalausbeute, bezogen auf eingesetztes Äthylenglykol von 53,9% der Theorie).

Vergleichsbeispiel (P allein im Kontakt).

Ein Gemisch aus Kupferpulver und Antimonphosphat (SbPO$_4$) wird durch Flammspritzen auf Steatitkugeln mit rauher Oberfläche von 6 mm Durchmesser aufgebracht. Der so hergestellte Katalysator enthält pro Liter Träger ca. 161 g aktive Masse der Zusammensetzung 88,6 Gewichtsprozent Cu; 0,5 Gewichtsprozent Sb und 0,097 Gewichtsprozent P. 63,03 Teile dieses Katalysators werden in einen aus V2A-Stahl konstruierten Röhrenreaktor mit 21 mm innerem Durchmesser eingebaut. Kontinuierlich wird stündlich ein gasförmiges Gemisch aus 6,75 Teilen Äthylenglykol, 2,16 Teilen Wasser, 2940 Volumenteilen Sauerstoff und 186 000 Volumenteilen Stickstoff über den bei 325°C gehaltenen Katalysator geleitet. Das Produktgemisch wird aus dem den Reaktor verlassenden Gasstrom mit Wasser ausgewaschen, und die so erhaltene wässrige Lösung nach Bilanzende analysiert.

Man erhält folgendes Ergebnis:
Eingesetzte Menge an Äthylenglykol
= 122,31 Teile
Wässrige Produkt-Lösung = 969,64 Teile
Die Produkt-Lösung enthält:
7,89 Gew.-% Glyoxal = 76,50 Teile
(entspricht einer Glyoxalausbeute, bezogen auf eingesetztes Äthylenglykol, von 66,9% der Theorie).

Nach einer weiteren, ununterbrochenen Laufzeit von 48 Stunden werden folgende Ergebnisse erhalten:

Eingesetzte Menge an Äthylenglykol
= 124,52 Teile
Wässrige Produkt-Lösung = 965,78 Teile
Die Produkt-Lösung enthält:
7,01 Gew.-% Glyoxal = 67,70 Teile
(entspricht einer Glyoxalausbeute, bezogen auf eingesetztes Äthylenglykol, von 58,1% der Theorie).

Beispiel 3

Entsprechend dem vorhergehenden Vergleichsbeispiel wird ein Katalysator durch Flammspritzen hergestellt. Dieser Katalysator enthält pro Liter Steatitträger ca. 179 g aktive Masse der Zusammensetzung 90,2 Gewichtsprozent Cu; 0,8 Gewichtsprozent Sb und 0,08 Gewichtsprozent P. 65,92 Teile des Katalysators werden in einen aus V2A-Stahl konstruierten Röhrenreaktor mit 21 mm innerem Durchmesser eingebaut. Kontinuierlich wird jetzt stündlich ein gasförmiges Gemisch aus 6,58 Teilen Äthylenglykol, 2,10 Teilen Wasser, 0,000196 Teilen Trimethylphosphat, (CH$_3$O)$_3$PO (das sind 6,59 ppm P, bezogen auf das Äthylenglykolgewicht), 3570 Volumenteilen Sauerstoff und 188500 Volumenteilen Stickstoff über den bei 335°C gehaltenen Katalysator geleitet. Das Produktgemisch wird aus dem den Reaktor verlassenden Gasstrom mit Wasser ausgewaschen, und die so erhaltene wässrige Lösung nach Bilanzende analysiert.

Folgende Ergebnisse werden erhalten:
Eingesetzte Menge an Äthylenglykol
= 188,16 Teile
Wässrige Produkt-Lösung = 439,71 Teile
Die Produkt-Lösung enthält:
17,44 Gew.-% Glyoxal = 76,69 Teile
(entspricht einer Glyoxalausbeute, bezogen auf eingesetztes Äthylenglykol, von 69,4% der Theorie).

Nach einer weiteren, beinahe ununterbrochenen Laufzeit von 2654 Stunden werden folgende Ergebnisse erhalten:

Eingesetzte Menge an Äthylenglykol
= 37,20 Teile
Wässrige Produkt-Lösung = 519,97 Teile
Die Produkt-Lösung enthält:
4,46 Gew.-% Glyoxal = 23,19 Teile
(entspricht einer Glyoxalausbeute, bezogen auf eingesetztes Äthylenglykol, von 66,7% der Theorie).

Beispiel 4

63,07 Teile des in Beispiel 3 beschriebenen Katalysators werden in einen aus V2A-Stahl konstruierten Röhrenreaktor mit 21 mm innerem Durchmesser eingebaut. Kontinuierlich wird jetzt stündlich ein gasförmiges Gemisch aus 6,65 Teilen Äthylenglykol, 2,12 Teilen Wasser, 0,000396 Teilen Trimethylphosphat (CH$_3$O)$_3$PO (das sind 13,2 ppm P, bezogen auf das Äthylenglykolgewicht), 3250 Volumenteile Sauerstoff und 187000 Volumenteilen Stickstoff über den bei 335°C gehaltenen Katalysator geleitet. Das Produktgemisch wird aus dem den Reaktor verlassenden Gasstrom mit Wasser ausgewaschen, und die so erhaltene wässrige Lösung nach Bilanzende analysiert.

Man erhält folgendes Ergebnis:
Eingesetzte Menge an Äthylenglykol
= 40,39 Teile
Wässrige Produkt-Lösung = 426,65 Teile
Die Produkt-Lösung enthält:
5,95 Gew.-% Glyoxal = 25,39 Teile
(entspricht einer Glyoxalausbeute, bezogen auf eingesetztes Äthylenglykol, von 67,2% der Theorie).

Nach einer weiteren, ununterbrochenen Laufzeit von 428 Stunden erhält man folgendes Ergebnis:
Eingesetzte Menge an Äthylenglykol
= 39,61 Teile
Wässrige Produkt-Lösung = 420,32 Teile
Die Produkt-Lösung enthält:
5,44 Gew.-% Glyoxal = 22,87 Teile
(entspricht einer Glyoxalausbeute, bezogen auf eingesetztes Äthylenglykol, von 61,7% der Theorie).

Beispiel 5

65,66 Teile des in Beispiel 3 beschriebenen Katalysators werden in einen aus V2A-Stahl konstruierten Röhrenreaktor mit 21 mm innerem Durchmesser eingebaut. Kontinuierlich wird jetzt stündlich ein gasförmiges Gemisch aus 6,47 Teilen Äthylenglykol, 2,06 Teilen Wasser, 0,000771 Teilen Trimethylphosphat $(CH_3O)_3PO$ (das sind 26,3 ppm P, bezogen auf das Äthylenglykolgewicht), 3250 Volumenteilen Sauerstoff und 187000 Volumenteilen Stickstoff über den bei 335°C gehaltenen Katalysator geleitet. Das Produktgemisch wird aus dem den Reaktor verlassenden Gasstrom mit Wasser ausgewaschen, und die so erhaltene wässrige Lösung nach Bilanzende analysiert.
Man erhält folgendes Ergebnis:
Eingesetzte Menge an Äthylenglykol
= 39,39 Teile
Wässrige Produkt-Lösung = 398,7 Teile
Die Produkt-Lösung enthält:
6,13 Gew.-% Glyoxal = 24.44 Teile
(entspricht einer Glyoxalausbeute, bezogen auf eingesetztes Äthylenglykol, von 66,4% Theorie).
Nach einer weiteren, ununterbrochenen Laufzeit von 221 Stunden wird folgendes Ergebnis erhalten:
Eingesetzte Menge an Äthylenglykol
= 40,67 Teile
Wässrige Produkt-Lösung = 409,41 Teile
Die Produkt-Lösung enthält:
5,52 Gew.-% Glyoxal = 22,60 Teile
(entspricht einer Glyoxalausbeute, bezogen auf eingesetztes Äthylenglykol, von 59,4% der Theorie).

Beispiel 6

64,7 Teile des in Beispiel 3 beschriebenen Katalysators werden in einen aus V2A-Stahl konstruierten Röhrenreaktor mit 21 mm innerem Durchmesser einbegaut. Kontinuierlich wird stündlich ein gasförmiges Gemisch aus 6,90 Teilen Äthylenglykol, 2,20 Teilen Wasser, 0,00206 Teilen Trimethylphosphat $(Ch_3O)_3PO$ (das sind 66 ppm P, bezogen auf das Äthylenglykolgewicht, 3250 Volumenteilen Sauerstoff und 177000 Volumenteilen Stickstoff über den bei 335°C gehaltenen Katalysator geleitet. Das Produktgemisch wird aus dem den Reaktor verlassenden Gasstrom mit Wasser ausgewaschen, und die so erhaltene wässrige Lösung nach Bilanzende analysiert.
Man erhält folgendes Ergebnis:
Eingesetzte Menge an Äthylenglykol
= 41,37 Teile
Wässrige Produkt-Lösung = 487,07 Teile
Die Produkt-Lösung enthält:

5,28 Gew.-% Glyoxal = 25,72 Teile
(entspricht einer Glyoxalausbeute, bezogen auf eingesetztes Äthylenglykol, von 66,5% der Theorie).
Nach einer weiteren, ununterbrochenen Laufzeit von 48 Stunden erhält man folgendes Ergebnis:
Eingesetzte Menge an Äthylenglykol
= 124,87 Teile
Wässrige Produkt-Lösung = 1110,88 Teile
Die Produkt-Lösung enthält:
6,34 Gew.-% Glyoxal = 70,34 Teile
(entspricht einer Glyoxalausbeute, bezogen auf eingesetztes Äthylenglykol, von 60,3% der Theorie).

**Patentansprüche**

1. Verfahren zur Herstellung von Glyoxal durch Dampfphasenoxidation von Ethylenglykol mit einem Sauerstoff enthaltenden Gas in Gegenwart von Kupfer enthaltenden Oxidationskatalysatoren bei erhöhter Temperatur, dadurch gekennzeichnet, dass man die Dampfphasenoxidation in Gegenwart einer unter den Reaktionsbedingungen gasförmigen Phosphorverbindung, mit Ausnahme von anorganischen Phosphorverbindungen, durchführt, wobei die Phosphormenge (berechnet P), bezogen auf die Gewichtsmenge an Ethylenglykol, 1 bis 100 ppm beträgt.

2. Verfahren nach Anspruch 1, dadurch gekennzeichnet, dass man die unter den Reaktionsbedingungen gasförmige Phosphorverbindung dem der Dampfphasenoxidation zuzuführenden Ethylenglykol zusetzt.

3. Verfahren nach Anspruch 1, dadurch gekennzeichnet, dass die Phosphormenge (berechnet P), bezogen auf die Gewichtsmenge an Ethylenglykol, 2 bis 25 ppm beträgt.

4. Verfahren nach Anspruch 1, dadurch gekennzeichnet, dass man als unter den Reaktionsbedingungen gasförmige Phosphorverbindung Trimethylphosphat, Triäthylphosphat, Triisopropylphosphat, Tri-n-propylphosphat, Trimethylphosphit, Triäthylphosphit, Triäthylphosphinoxid, Methylphosphonsäurediäthylester, Methylphosphonsäuredimethylester oder Äthylphosphonsäurediäthylester verwendet.

**Claims**

1. A process for the preparation of glyoxal by vapor phase oxidation of ethylene glycol with an oxygen-containing gas in the presence of a copper-containing oxidation catalyst at an elevated temperature, wherein the vapor phase oxidation is carried out in the presence of a phosphorus compound which is gaseous under the reaction conditions, excluding inorganic phosphorus compounds, the amount of phosphorus (calculated as P) being from 1 to 100 ppm, based on the weight of ethylene glycol.

2. A process as claimed in claim 1, wherein the phosphorus compound which is gaseous under the reaction conditions is added to the ethylene glycol to be fed to the vapor phase oxidation.

3. A process as claimed in claim 1, wherein the amount of phosphorus (calculated as P) is from 2 to 25 ppm, based on the weight of ethylene glycol.

4. A process as claimed 1, wherein trimethyl phosphate, triethyl phosphate, tri-isopropyl phosphate, tri-n-propyl phosphate, trimethyl phosphite, triethyl phosphite, triethylphosphine oxide, diethyl methylphosphonate, dimethyl methylphosphonate or diethyl ethylphosphonate is used as the phosphorus compound which is gaseous under the reaction conditions.

**Revendications**

1. Procédé de préparation du glyoxal par une oxydation de l'éthylène-glycol en phase vapeur, à température accrue et en présence de catalyseurs d'oxydation au cuivre, par un gaz contenant de l'oxygène, caractérisé en ce que l'oxydation en phase vapeur est réalisée en présence d'un composé de phosphore gazeux dans les conditions réactionnelles, à l'exception des dérivés du phosphore inorganiques, la proportion du phosphore (calculé en P) par rapport à la proportion pondérale de l'éthylène-glycol étant de 1 à 100 ppm.

2. Procédé suivant la revendication 1, caractérisé en ce que le composé de phosphore gazeux dans les conditions opératoires est additionné à l'éthylène-glycol à soumettre à l'oxydation en phase vapeur.

3. Procédé suivant la revendication 1, caractérisé en ce que la proportion du phosphore (calculé en P) par rapport à la proportion pondérale de l'éthylène-glycol est de 2 à 25 ppm.

4. Procédé suivant la revendication 1, caracétrisé en ce que le composé de phosphore gazeux dans les conditions réactionnelles est choisi parmi le phosphate de triméthyle, le phosphate de triéthyle, le phosphate de triisopropyle, le phosphate de tri-n-propyle, le phosphite de triméthyle, le phosphite de triéthyle, l'oxyde de triéthyl-phosphine, l'ester diéthylique de l'acide méthyl-phosphonique, l'ester diméthylique de l'acide méthyl-phosphonique et l'ester diéthylique de l'acide éthyl-phosphonique.